# EUROPEAN PATENT APPLICATION

(11) **EP 3 293 174 A1**
(43) Date of publication of application: **14.03.2018**
(21) Application number: 16188152.9
(22) Date of filing: 09.09.2016
(51) Int. Cl.: C07D 213/75, A61K 31/44, A61P 7/02

(54) **CRYSTALLINE SALTS OF BETRIXABAN**

(71) Applicant: SANDOZ AG, 4056 Basel (CH)
(72) Inventor: MARTIN, Nolwenn, 6250 Kundl (AT)
(74) Representative: Prüfer & Partner mbB Patentanwälte · Rechtsanwälte

(57) **Abstract**

The present invention relates to crystalline salts of betrixaban and processes for their preparation. More specifically, it relates to crystalline betrixaban hydrochloride, crystalline betrixaban citrate and to crystalline form B of betrixaban hydrobromide. It also relates to a pharmaceutical composition comprising said crystalline salts of betrixaban and one or more pharmaceutically acceptable excipients. The invention also concerns the use of said pharmaceutical compositions as a medicament, in particular for the prevention and the treatment of disease conditions characterized by undesired thrombosis.

## Description

### FIELD OF THE INVENTION

The present invention relates to crystalline salts of betrixaban and processes for their preparation. It also relates to a pharmaceutical composition comprising said crystalline salts of betrixaban and one or more pharmaceutically acceptable excipients. The invention also concerns the use of said pharmaceutical compositions as a medicament, in particular for the prevention and the treatment of disease conditions characterized by undesired thrombosis.

### BACKGROUND OF THE INVENTION

Betrixaban, N-(5-chloropyridin-2-yl)-2-([4-(N,N-dimethylcarbamimidoyl)benzoyl]amino)-5-methoxybenzamide, is a Factor Xa inhibitor under clinical development for the prevention and treatment of venous thrombosis. It can be represented by the following chemical structure:

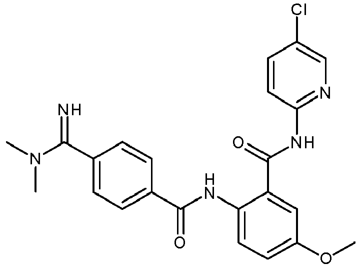

WO 2007/056517 discloses betrixaban salts and a process for their preparation. In particular betrixaban maleate (1:1) and a crystalline polymorphic form thereof called form I is disclosed. Such form suffers from a poor water solubility of about 2.05 to about 2.25 mg/mL. It also discloses several other salts of betrixaban, which are however characterized by poor crystallinity and/or poor hydrolytic stability.

WO 2012/031017 discloses two further crystalline forms of betrixaban maleate: form II - the thermodynamically most stable form - and form III, which is a channel hemihydrate of form II. Form II was first produced inadvertently while attempting to make form I and reversibly converts to form III at relative humidity above 25%. The process for the preparation of form I is therefore not reliable and crystalline forms II and III are moisture sensitive since they reversible convert to each other depending on the relative humidity. There are therefore several drawbacks related to the reliability of the preparation of betrixaban maleate form I - the thermodynamically less stable form - and to the physical stability of the forms II and III depending on the relative humidity.

WO 2015/176591 discloses several salts of betrixaban and their crystal forms, which are allegedly more controllable, stable and soluble than the crystalline forms of betrixaban maleate known in the art, e.g. in WO 2012/031017. However, no information about the physical stability of the salts (e.g. physical stability under accelerated storage conditions, at elevated relative humidity, at elevated temperature) is provided.

Hence there is a need for a crystalline salt of betrixaban with improved physicochemical properties, which can be prepared with a reliable industrial process; there is a particular need for thermodynamically stable, highly water soluble and non-hygroscopic crystalline forms of betrixaban salts with improved behaviour upon contact with moisture or under drying conditions.

### SUMMARY OF THE INVENTION

The present inventors have realized that some betrixaban salts prepared with "Generally Recognized As Safe" (GRAS) acids, such as hydrochloric acid or citric acid, have superior physicochemical properties (e.g. superior thermodynamic and moisture stabilities) in respect to salts of betrixaban prepared with non-GRAS acid such as maleic acid. These advantageous features can be beneficial for the prevention and the long-term treatment of disease conditions characterized by undesired thrombosis.

Therefore, the present invention relates to crystalline salts of betrixaban and processes for their preparation. More specifically, it relates to crystalline betrixaban hydrochloride, and in particular to crystalline form A and form B of betrixaban hydrochloride, and to crystalline betrixaban citrate, and in particular to crystalline form A of betrixaban citrate. It also relates to a crystalline form B of betrixaban hydrobromide.

It also relates to a pharmaceutical composition comprising said crystalline salts of betrixaban and one or more pharmaceutically acceptable excipients. In addition, the present invention relates to the use of said pharmaceutical composition as a medicament, in particular for the treatment of disease conditions characterized by undesired thrombosis.

### Abbreviations

- PXRD: powder X-ray diffractogram
- DSC: differential scanning calorimetry
- TGA: thermogravimetric analysis
- GMSD: gravimetric moisture sorption/ desorption
- Δm: mass difference
- RH: relative humidity

### Definitions

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to PXRD means that variabilities in peak positions and relative intensities of the peaks are to be taken into account. For example, a typical precision of the 2-Theta values is in the range of ± 0.2° 2-Theta. Thus, a diffraction the peak of form A that usually appears at 9.3° 2-Theta for example can appear between 9.1° and 9.5° 2-Theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

The term "essentially the same" with reference to infrared spectrometry means that variabilities in peak positions and relative intensities of the peaks are to be taken into account. For example, a typical precision of the wavenumber values is in the range of ± 2 cm⁻¹. Thus, the peak that usually appears at 1668 cm⁻¹ for example can appear between 1666 and 1670 cm⁻¹ on most IR-spectrometers under standard conditions. Differences in relative intensities are typically smaller compared to X-ray diffraction. However, one skilled in the art will appreciate that small differences in peak intensities due to degree of crystallinity, sample preparation and other factors can also occur in IR-spectroscopy. Bigger intensity differences will occur when IR spectra are recorded in transmission (KBr pellet or Nujol mull) instead of attenuated total reflectance (ATR). Relative peak intensities should therefore be taken as qualitative measure only.

As used herein, the term "about" means within a statistically meaningful range of a value. Such a range can be within an order of magnitude, typically within 10%, more typically within 5%, even more typically within 1% and most typically within 0.5% of the indicated value or range. Sometimes, such a range can lie within the experimental error, typical of standard methods used for the measurement and/or determination of a given value or range.

The terms "solvate" or "hydrate" as used herein refers to a solid, where organic solvent or water, respectively, is coordinated in or accommodated by the crystal structure.

As used herein, the term "substantially pure" with reference to a particular physical form means that the physical form includes at most 20%, preferably at most 10%, more preferably at most 5%, even more preferably at most 2% and most preferably at most 1% by weight of any other physical form.

As used herein, the term "betrixaban hydrobromide form A" refers to crystalline betrixaban hydrobromide characterized by having a powder X-ray diffraction pattern comprising peaks at 2-theta angles of 5.8 ± 0.2°, 6.5 ± 0.2°, 8.8 ± 0.2°, 11.7 ± 0.2°, 15.1 ± 0.2°, 17.8 ± 0.2°, 21.3 ± 0.2°, 21.7 ± 0.2°, 22.3 ± 0.2°, 25.3 ± 0.2° and 26.7 ± 0.2°, when measured at ambient temperature (i.e. 0-40°C) with Cu-Kα radiation (according to WO 2015/176591).

### BRIEF DESCRIPTION OF THE FIGURES

**Figure 1****:** Representative PXRD pattern of betrixaban hydrochloride form A prepared according to the procedure described in example 1-1. The X-axis shows the 2-theta angle / ° and the Y-axis shows the intensity / counts.
**Figure 2****:** Representative FTIR spectrum of betrixaban hydrochloride form A prepared according to the procedure described in example 1-1. The X-axis shows the wavenumber / cm⁻¹ and the Y-axis shows the transmittance / %.
**Figure 3****:** Representative DSC curve of betrixaban hydrochloride form A prepared according to the procedure described in example 1-1. The X-axis shows the temperature / °C and the Y-axis shows peak heights / Wg⁻¹. Endothermic events are plotted up.
**Figure 4****:** Representative TGA curve of betrixaban hydrochloride form A prepared according to the procedure described in example 1-1. The X-axis shows the temperature / °C and the Y-axis shows sample mass / %. Endothermic events are plotted up.
**Figure 5****:** Representative gravimetric moisture sorption/ desorption isotherms of betrixaban hydrochloride form A. The X-axis shows the relative humidity / % and the Y-axis shows the mass difference Δm / %. The sorption isotherm is represented by the square symbols (symbols: ■); the desorption isotherm is represented by the sphere symbols (symbols: ●).
**Figure 6****:** Representative PXRD of betrixaban hydrochloride form B prepared according to the procedure described in example 2. The X-axis shows the 2-theta angle / ° and the Y-axis shows the intensity / counts.
**Figure 7****:** Representative PXRD pattern of crystalline form A of betrixaban citrate prepared according to the procedure described in example 3-1. The X-axis shows the 2-theta angle / ° and the Y-axis shows the intensity / counts.
**Figure 8****:** Representative gravimetric moisture sorption/ desorption isotherms of crystalline form A of betrixaban citrate prepared according to the procedure described in example 3-1. The X-axis shows the relative humidity / % and the Y-axis shows the mass difference Δm / %. The sorption isotherm is represented by the square symbols (symbols: ■); the desorption isotherm is represented by the sphere symbols (symbols: ●).
**Figure 9****:** Representative PXRD pattern of crystalline form B of betrixaban hydrobromide prepared according to the procedure described in example 4-1. The X-axis shows the 2-theta angle / ° and the Y-axis shows the intensity / counts.
**Figure 10****:** Representative gravimetric moisture sorption/ desorption isotherms of crystalline form B of betrixaban hydrobromide prepared according to the procedure described in example 4-1. The X-axis shows the relative humidity / % and the Y-axis shows the mass difference Δm / %. The sorption isotherm is represented by the square symbols (symbols: ■); the desorption isotherm is represented by the sphere symbols (symbols: ●).

### DETAILED DESCRIPTION OF THE INVENTION

### Betrixaban hydrochloride form A

In a first aspect, the present invention relates to crystalline betrixaban hydrochloride and more particularly to crystalline forms of betrixaban hydrochloride, hereinafter also designated as form A and form B of betrixaban hydrochloride. Form A of betrixaban hydrochloride is prepared using an easy and reliable process and can further be used for the preparation of form B of betrixaban hydrochloride, which is thermodynamically more stable.

In one embodiment, the present invention relates to crystalline form A of betrixaban hydrochloride, a crystalline form which can be characterized by having a PXRD comprising peaks at 2-Theta angles of 9.3 ± 0.2°, 12.1 ± 0.2°, 13.8 ± 0.2°, 14.5 ± 0.2° and 25.0 ± 0.2°, when measured at room temperature using Cu-Kα_{1,2} radiation having a wavelength of 0.15419 nm. The PXRD of crystalline form A of betrixaban hydrochloride may be further characterized by comprising one or more additional peaks, double peaks and/or multiple peaks at 2-Theta angles of 11.6 ± 0.2°, 16.4 ± 0.2°, 18.8 ± 0.2°, 22.6 ± 0.2°, 22.9 ± 0.2°, 23.5 ± 0.2°, 23.8 ± 0.2°, 24.4 ± 0.2°, 26.9 ± 0.2° and/or 28.6 ± 0.2°, when measured at room temperature with Cu-Kα_{1,2} radiation having a wavelength of 0.15419 nm.

Alternatively or additionally, crystalline form A of betrixaban hydrochloride can be characterized by having a PXRD comprising five or more peaks at 2-Theta angles of 9.3 ± 0.2°, 11.6 ± 0.2°, 12.1 ± 0.2°, 13.8 ± 0.2°, 14.5 ± 0.2°, 16.4 ± 0.2°, 18.8 ± 0.2°, 22.6 ± 0.2°, 22.9 ± 0.2°, 23.5 ± 0.2°, 23.8 ± 0.2°, 24.4 ± 0.2°, 25.0 ± 0.2°, 26.9 ± 0.2° and/or 28.6 ± 0.2°, when measured at room temperature with Cu-Kα1,2 radiation having a wavelength of 0.15419 nm.

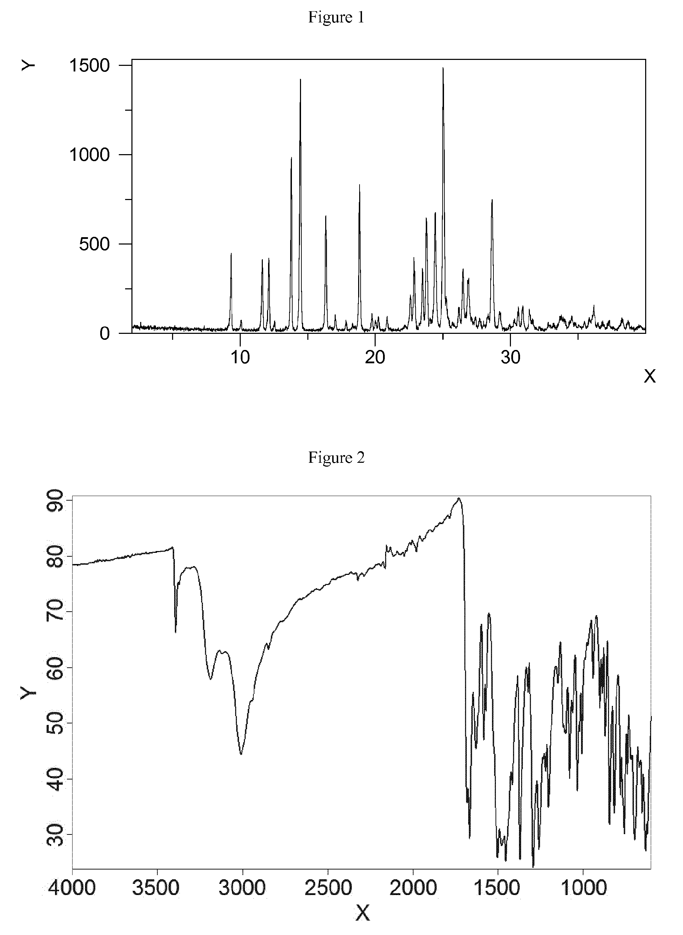

Alternatively or additionally, crystalline form A of betrixaban hydrochloride can be characterized by having a PXRD essentially the same as displayed in figure 1, when measured at room temperature with Cu-Kα_{1,2} radiation having a wavelength of 0.15419 nm.

Alternatively or additionally, crystalline form A of betrixaban hydrochloride can be characterized by having an FTIR spectrum comprising characteristic peaks, double peaks and/or multiple peaks at wavenumbers of 3394 ± 2 cm⁻¹, 3189 ± 2 cm⁻¹, 3011 ± 2 cm⁻¹, 1668 ± 2 cm⁻¹ and 1294 ± 2 cm⁻¹ when measured at room temperature with a ZnSe ATR cell. The FTIR spectrum of crystalline form A of betrixaban hydrochloride may be further characterized by comprising one or more additional peaks, double peaks and/ or multiple peaks at wavenumbers of 1504 ± 2 cm⁻¹, 1456 ± 2 cm⁻¹, 1372 ± 2 cm⁻¹, 1261 ± 2 cm⁻¹ and/ or 846± 2 cm⁻¹, when measured at room temperature with a ZnSe ATR cell.

Alternatively or additionally, crystalline form A of betrixaban hydrochloride can be characterized by having a FTIR spectrum essentially the same as displayed in figure 2, when measured at room temperature with a ZnSe ATR cell.

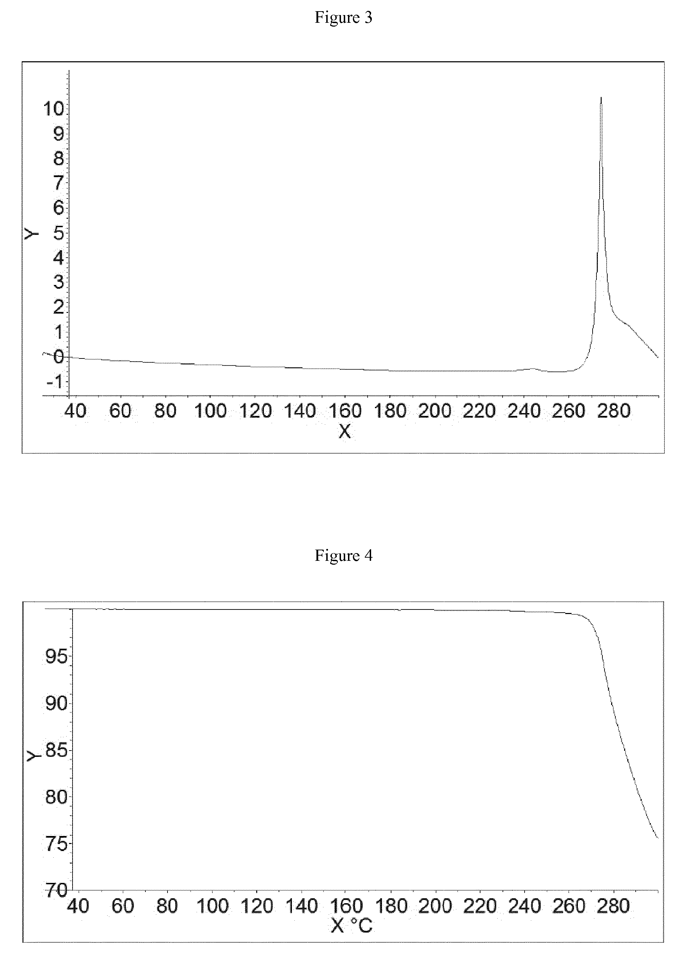

Furthermore, a representative DSC curve of crystalline form A of betrixaban hydrochloride is displayed in figure 3. The DSC curve of crystalline form A of betrixaban hydrochloride shows a first endothermic event with an onset temperature of about 232°C and a peak maximum at about 244°C corresponding to the transformation of crystalline form A to crystalline form B, followed by a melting endotherm with an onset temperature of about 272°C and a peak maximum at about 273°C.

Therefore, alternatively or additionally, crystalline form A of betrixaban hydrochloride can be characterized by a DSC curve comprising a first endothermic event with an onset temperature of about 232°C and a peak maximum at about 244°C, followed by a second endotherm with an onset temperature of about 272°C and a peak maximum at about 273°C, when measured at a heating rate of 10°C/min.

Alternatively or additionally, crystalline form A of betrixaban hydrochloride can be characterized by transforming to polymorphic form B at a temperature of about 232-244°C when measured with DSC at a heating rate of 10°C/min.

Alternatively or additionally, crystalline form A of betrixaban hydrochloride can be characterized by having a DSC curve essentially the same as displayed in figure 3.

In addition, a representative TGA curve of crystalline form A of betrixaban hydrochloride is provided in figure 4 herein. The TGA curve of form A of betrixaban hydrochloride shows no significant weight loss from 25 to about 230°C - proofing the absence of volatile components such as residual organic solvents - and a weight loss of about 0.4% from about 230 to 255°C. Hence, form A can be characterized as being a non-solvated form of betrixaban hydrochloride.

Alternatively or additionally, crystalline form A of betrixaban hydrochloride can be characterized by comprising about ≤ 1.0%, preferably about ≤ 0.8%, more preferably about ≤ 0.6% and most preferably about ≤ 0.4% organic solvent.

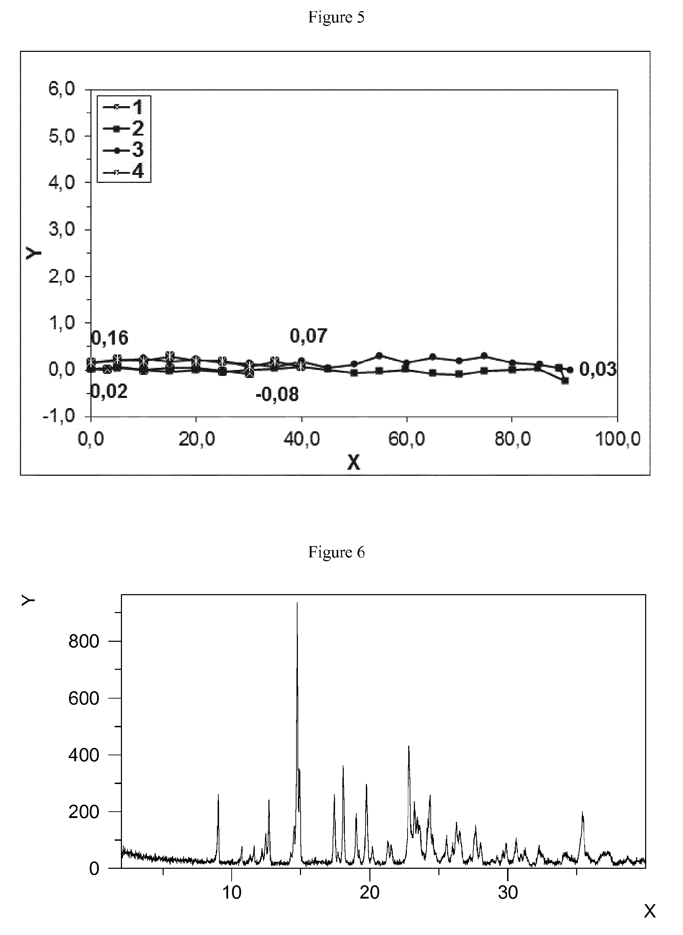

Moreover, representative gravimetric moisture sorption/desorption (GMSD) isotherms of crystalline form A of betrixaban hydrochloride measured at 25 ± 0.1°C are displayed in figure 5 herein. Form A, for example, shows a water content of from 0.0 to 0.2% at about 0% RH and a water content of from 0.0 to 0.2% at about 95% RH. According to the GMSD isotherms displayed in figure 5 crystalline form A of betrixaban hydrochloride is a non-hygroscopic anhydrate of betrixaban hydrochloride. Hence, alternatively or additionally, crystalline form A of betrixaban hydrochloride can be characterized as being an anhydrate.

Alternatively or additionally, crystalline form A of betrixaban hydrochloride can be characterized by comprising ≤ 0.2% water when measured at 0 to 95% RH and 25 ± 0.1°C.

Alternatively or additionally, crystalline form A of betrixaban hydrochloride can be characterized by being stable upon storage under normal conditions, preferably under storage at a relative humidity of 45% at 23°C for at least two six months, more preferably for at least one year, most preferably for at least two years. Alternatively or additionally, crystalline form A of betrixaban hydrochloride can be characterized by being stable upon storage under accelerated conditions, preferably under storage at a relative humidity of 70% at 40°C for at least one month, more preferably for at least three months, most preferably for at least six months.

In a preferred embodiment, crystalline form A of betrixaban hydrochloride is in substantially pure form.

### Betrixaban hydrochloride form B

In a second embodiment, the present invention relates to crystalline form B of betrixaban hydrochloride, a crystalline form which can be characterized by having a PXRD comprising peaks at 2-Theta angles of 9.0 ± 0.2°, 12.7 ± 0.2°, 14.7 ± 0.2°, 18.1 ± 0.2° and 22.8 ± 0.2°, when measured at room temperature using Cu-Kα_{1,2} radiation having a wavelength of 0.15419 nm. The PXRD of form B may be further characterized by comprising one or more additional peaks, double peaks and/ or multiple peaks at 2-Theta angles of 12.5 ± 0.2°, 14.5 ± 0.2°, 14.9 ± 0.2°, 17.4 ± 0.2°, 19.0 ± 0.2°, 19.8 ± 0.2°, 23.2 ± 0.2°, 23.4 ± 0.2°, 24.4 ± 0.2°, 26.3 ± 0.2°, 26.5 ± 0.2° and/or 27.7 ± 0.2°, when measured at room temperature with Cu-Kα_{1,2} radiation having a wavelength of 0.15419 nm.

Alternatively or additionally, crystalline form B of betrixaban hydrochloride can be characterized by having a PXRD comprising five or more peaks at 2-Theta angles of 9.0 ± 0.2°, 12.5 ± 0.2°, 12.7 ± 0.2°, 14.5 ± 0.2°, 14.7 ± 0.2°, 14.9 ± 0.2°, 17.4 ± 0.2°, 18.1 ± 0.2°, 19.0 ± 0.2°, 19.8 ± 0.2°, 22.8 ± 0.2°, 23.2 ± 0.2°, 23.4 ± 0.2°, 24.4 ± 0.2°, 26.3 ± 0.2°, 26.5 ± 0.2° and/or 27.7 ± 0.2°, when measured at room temperature with Cu-Kα_{1,2} radiation having a wavelength of 0.15419 nm.

Alternatively or additionally, crystalline form B of betrixaban hydrochloride can be characterized by having a PXRD essentially the same as displayed in figure 6 of the present invention, when measured at room temperature with Cu-Kα_{1,2} radiation having a wavelength of 0.15419 nm.

Furthermore, as displayed in the DSC curve of crystalline form A of betrixaban hydrochloride in figure 3 herein, crystalline form A transforms to crystalline form B at onset temperature of about 232°C and a peak maximum at about 244°C (endothermic event), followed by a melting endotherm of crystalline form B with an onset temperature of about 272°C and a peak maximum at about 273°C.

Therefore, alternatively or additionally, crystalline form B of betrixaban hydrochloride can be characterized by having a melting point of from about 272 to 273°C when measured with DSC at a heating rate of 10°C/min.

Additionally, crystalline form B of betrixaban hydrochloride can be characterized as being a non-solvated form of betrixaban hydrochloride.

Alternatively or additionally, crystalline form B of betrixaban hydrochloride can be characterized by comprising about ≤ 1.0%, preferably about ≤ 0.8%, more preferably about ≤ 0.6% and most preferably about ≤ 0.4% organic solvent or water.

Alternatively or additionally, crystalline form B of betrixaban hydrochloride can be characterized by being stable upon storage under normal conditions, preferably under storage at a relative humidity of 45% at 23°C for at least two six months, more preferably for at least one year, most preferably for at least two years. Alternatively or additionally, crystalline form B of betrixaban hydrochloride can be characterized by being stable upon storage under accelerated conditions, preferably under storage at a relative humidity of 70% at 40°C for at least one month, more preferably for at least three months, most preferably for at least six months.

In a preferred embodiment, crystalline form B of betrixaban hydrochloride is in substantially pure form.

### Betrixaban citrate

In a third embodiment, the present invention relates to betrixaban citrate, and in particular to crystalline form A of betrixaban citrate, which can be characterized by having a PXRD comprising peaks at 2-Theta angles of 6.2 ± 0.2°, 8.1 ± 0.2°, 12.7 ± 0.2°, 14.7 ± 0.2° and 25.7 ± 0.2°, when measured at room temperature using Cu-Kα_{1,2} radiation having a wavelength of 0.15419 nm. The PXRD of the crystalline form A of betrixaban citrate may be further characterized by comprising one or more additional peaks, double peaks and/ or multiple peaks at 2-Theta angles of 7.1 ± 0.2°, 13.1 ± 0.2°, 15.9 ± 0.2°, 17.0 ± 0.2°, 18.1 ± 0.2°, 18.3 ± 0.2°, 20.6 ± 0.2°, 21.1 ± 0.2°, 23.3 ± 0.2°, 23.6 ± 0.2° and/or 25.2 ± 0.2°, when measured at room temperature with Cu-Kα_{1,2} radiation having a wavelength of 0.15419 nm.

Alternatively or additionally, crystalline form A of betrixaban citrate can be characterized by having a PXRD comprising five or more peaks at 2-Theta angles of 6.2 ± 0.2°, 7.1 ± 0.2°, 8.1 ± 0.2°, 12.7 ± 0.2°, 13.1 ± 0.2°, 14.7 ± 0.2°, 15.9 ± 0.2°, 17.0 ± 0.2°, 18.1 ± 0.2°, 18.3 ± 0.2°, 20.6 ± 0.2°, 21.1 ± 0.2°, 23.3 ± 0.2°, 23.6 ± 0.2°, 25.2 ± 0.2° and/or 25.7 ± 0.2° when measured at room temperature with Cu-Kα_{1,2} radiation having a wavelength of 0.15419 nm.

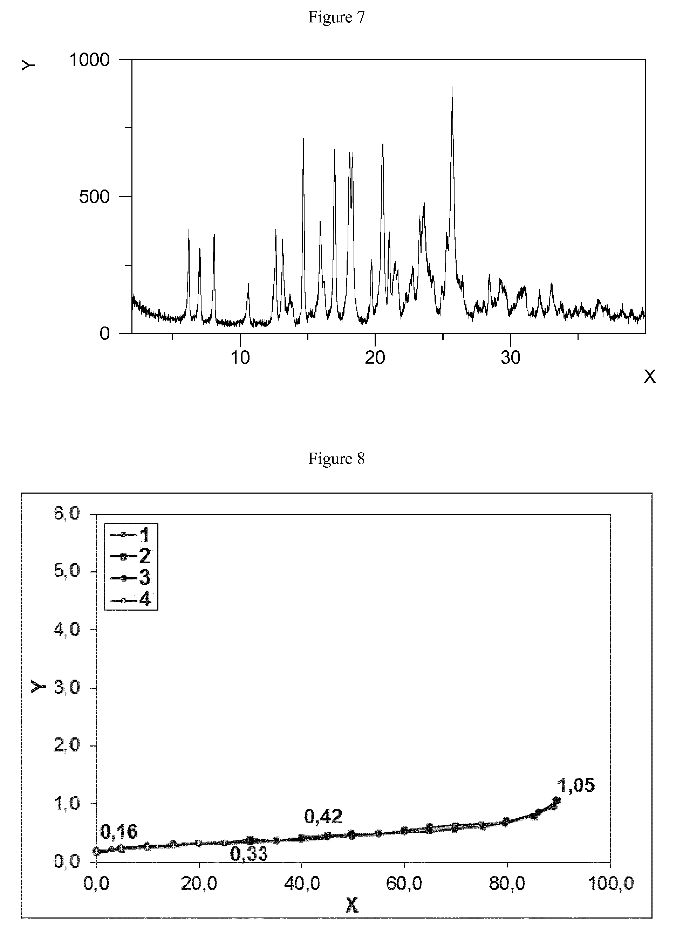

Alternatively or additionally, crystalline form A of betrixaban citrate can be characterized by having a PXRD essentially the same as displayed in figure 7 herein, when measured at room temperature with Cu-Kα_{1,2} radiation having a wavelength of 0.15419 nm.

Furthermore, a representative DSC curve of crystalline form A of betrixaban citrate shows a melting endotherm with an onset temperature of about 166°C and a peak maximum at about 168°C.

Therefore, alternatively or additionally, crystalline form A of betrixaban citrate can be characterized by a DSC curve comprising an endotherm with an onset temperature of about 166°C and a peak maximum at about 168°C when measured at a heating rate of 10°C/min. Alternatively or additionally, crystalline form A of betrixaban citrate can be characterized by having a melting point of about 166 to 168°C when measured with DSC at a heating rate of 10°C/min.

In addition, a representative TGA curve of crystalline form A of betrixaban citrate shows a continuous weight loss of less than 1.0% from 25 to 166°C, corresponding to non-bounded residual solvent. No modification of the physical form of crystalline form A of betrixaban citrate is observed from 25 to 166°C. Hence, crystalline form A of betrixaban citrate can be characterized as being a non-solvated form of betrixaban citrate.

Moreover, representative gravimetric moisture sorption/desorption (GMSD) isotherms of the crystalline form A of betrixaban citrate measured at 25 ± 0.1°C are displayed in figure 8 herein. Crystalline form A of betrixaban citrate, for example, shows a water content of from 0.0 to 0.2% at about 0% RH and a water content of from 1.0 to 1.1% at about 95% RH. According to the GMSD isotherms displayed in figure 8, crystalline form A of betrixaban citrate is an anhydrate of betrixaban citrate being slightly hygroscopic. Hence, alternatively or additionally, crystalline form A of betrixaban citrate can be characterized as being an anhydrate.

Alternatively or additionally, crystalline form A of betrixaban citrate can be characterized by comprising ≤ 1.1% water when measured at 0 to 95% RH and 25 ± 0.1 °C.

Alternatively or additionally, crystalline form A of betrixaban citrate can be characterized by being stable upon storage under normal conditions, preferably under storage at a relative humidity of 45% at 23°C for at least two six months, more preferably for at least one year, most preferably for at least two years. Alternatively or additionally, crystalline form A of betrixaban citrate can be characterized by being stable upon storage under accelerated conditions, preferably under storage at a relative humidity of 70% at 40°C for at least one month, more preferably for at least three months, most preferably for at least six months.

In a preferred embodiment, crystalline form A of betrixaban citrate is in substantially pure form.

### Betrixaban hydrobromide form B

In a fourth embodiment, the present invention relates to crystalline form B of betrixaban hydrobromide, which can be characterized by having a PXRD comprising peaks at 2-Theta angles of 12.5 ± 0.2°, 13.6 ± 0.2°, 16.0 ± 0.2°, 18.8 ± 0.2° and 24.6 ± 0.2°, when measured at room temperature using Cu-Kα_{1,2} radiation having a wavelength of 0.15419 nm. The PXRD of crystalline form B of betrixaban hydrobromide may be further characterized by comprising one or more additional peaks, double peaks and/or multiple peaks at 2-Theta angles of 11.6 ± 0.2°, 13.7 ± 0.2°, 15.0 ± 0.2°, 16.6 ± 0.2°, 20.4 ± 0.2°, 22.0 ± 0.2°, 23.0 ± 0.2°, 23.5 ± 0.2°, 23.8 ± 0.2°, 25.0 ± 0.2° and/ or 25.3 ± 0.2°, when measured at room temperature with Cu-Kα_{1,2} radiation having a wavelength of 0.15419 nm.

Alternatively or additionally, crystalline form B of betrixaban hydrobromide can be characterized by having a PXRD comprising no significant peak at 2-Theta angles of 2.0 to 9.5°, when measured at room temperature with Cu-Kα1,2 radiation having a wavelength of 0.15419 nm. The term "no significant peak" with reference to PXRD means that no peak or peak with an intensity lower than 1% appear in the described range of 2-Theta angles.

Alternatively or additionally, crystalline form B of betrixaban hydrobromide can be characterized by having a PXRD comprising no significant peaks at 2-Theta angles of 2.0 to 9.5° and comprising five or more peaks at 2-Theta angles selected from 11.6 ± 0.2°, 12.5 ± 0.2°, 13.6 ± 0.2°, 13.7 ± 0.2°, 15.0 ± 0.2°, 16.0 ± 0.2°, 16.6 ± 0.2°, 18.8 ± 0.2°, 20.4 ± 0.2°, 22.0 ± 0.2°, 23.0 ± 0.2°, 23.5 ± 0.2°, 23.8 ± 0.2°, 24.6 ± 0.2°, 25.0 ± 0.2° and/or 25.3 ± 0.2°, when measured at room temperature with Cu-Kα1,2 radiation having a wavelength of 0.15419 nm.

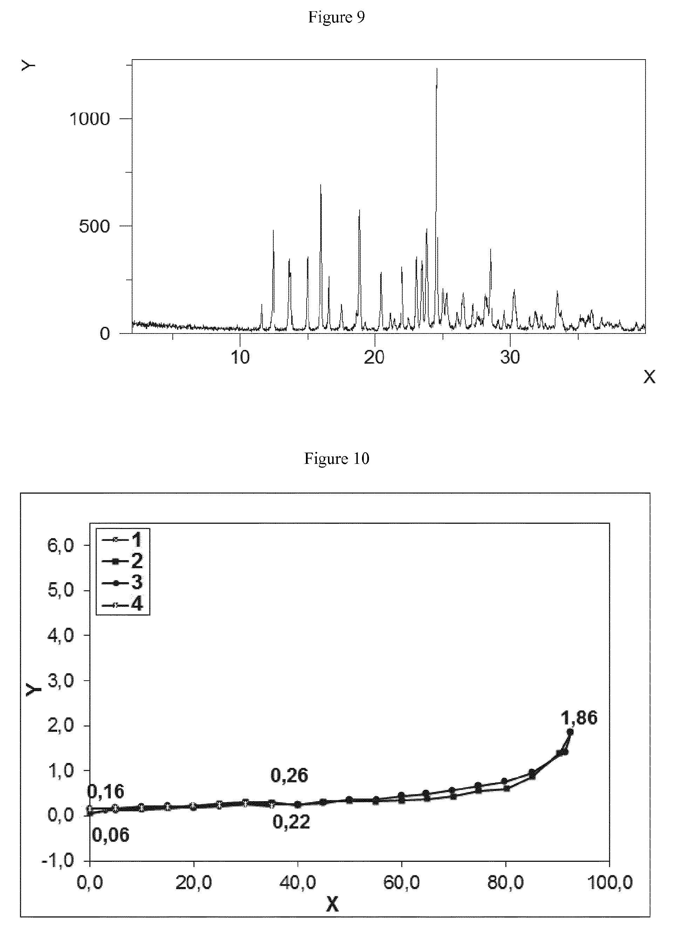

Alternatively or additionally, crystalline form B of betrixaban hydrobromide can be characterized by having a PXRD essentially the same as displayed in figure 9 herein, when measured at room temperature with Cu-Kα_{1,2} radiation having a wavelength of 0.15419 nm.

Furthermore, a representative DSC curve of crystalline form B of betrixaban hydrobromide shows a melting endotherm with an onset temperature of about 246°C and a peak maximum at about 249°C. Therefore, alternatively or additionally, crystalline form B of betrixaban hydrobromide can be characterized by a DSC curve comprising an endotherm with an onset temperature of about 246°C and a peak maximum at about 249°C when measured at a heating rate of 10°C/min.

Alternatively or additionally, crystalline form B of betrixaban hydrobromide can be characterized by having a melting point of about 246 to 249°C when measured with DSC at a heating rate of 10°C/min.

In addition, a representative TGA curve of crystalline form B of betrixaban hydrobromide shows a continuous weight loss of less than 1.5% from 25 to 246°C, corresponding to non-bounded residual solvent. No modification of the physical form of crystalline form B of betrixaban hydrobromide is observed from 25 to 246°C. Hence, crystalline form B of betrixaban hydrobromide can be characterized as being a non-solvated form of betrixaban hydrobromide.

Moreover, representative gravimetric moisture sorption/desorption (GMSD) isotherms of crystalline form B of betrixaban hydrobromide measured at 25 ± 0.1 °C are displayed in figure 10 herein. Crystalline form B of betrixaban hydrobromide, for example, shows a water content of from 0.0 to 0.2% at about 0% RH and a water content of from 1.8 to 1.9% at about 95% RH. According to the GMSD isotherms displayed in figure 10, crystalline form B of betrixaban hydrobromide is an anhydrate of betrixaban hydrobromide being moderately hygroscopic. Hence, alternatively or additionally, crystalline form B of betrixaban hydrobromide can be characterized as being an anhydrate.

Alternatively or additionally, crystalline form B of betrixaban hydrobromide can be characterized by comprising ≤ 1.9% water when measured at 0 to 95% RH and 25 ± 0.1°C.

Alternatively or additionally, crystalline form B of betrixaban hydrobromide can be characterized by being stable upon storage under normal conditions, preferably under storage at a relative humidity of 45% at 23°C for at least two six months, more preferably for at least one year, most preferably for at least two years. Alternatively or additionally, crystalline form B of betrixaban hydrobromide can be characterized by being stable upon storage under accelerated conditions, preferably under storage at a relative humidity of 70% at 40°C for at least one month, more preferably for at least three months, most preferably for at least six months.

In a preferred embodiment, crystalline form B of betrixaban hydrobromide is in substantially pure form. Preferably, crystalline form B of betrixaban hydrobromide includes less than 10%, more preferably less than 5%, even more preferably less than 3%, most preferably less than 1% by weight of crystalline form A of betrixaban hydrobromide.

### General process for the preparation of betrixaban salts

The present invention relates to a process for the preparation of the crystalline salts of betrixaban of the invention comprising the steps of
i) providing betrixaban free base
ii) (a) adding at least one organic solvent to obtain a suspension and (b) adding the acid,
iii) optionally seeding the mixture with the crystalline salt of betrixaban and
iv) subjecting the mixture provided in (iii) to crystallization conditions leading to the formation of the crystalline salt of betrixaban
v) optionally isolating the crystalline salt of betrixaban, and
vi) optionally drying the crystalline salt of betrixaban

In step (i) any physical form of betrixaban may be used, such as crystalline, amorphous form, or a mixture of thereof. Betrixaban can for example be prepared in accordance with the procedures disclosed in WO2001/19788, example 266.

In step (ii) the solvent is added at a temperature in the range of from 10 to 40°C, more preferably in the range of from 20 to 30°C, preferably at ambient pressure. In step (iib) the mole ratio of betrixaban and acid is preferably in the range of from 1.0:1.0 to 1.0:1.5, more preferably in the range of from 1.0:1.0 to 1.0:1.3.

Optionally, seed crystals may be added in step (iii). The seed crystals are prepared with the same solvent as the one used in step (iia) of the process. In particular, the seed crystals are prepared using the same process steps as the specific crystalline salt of betrixaban of the present invention. The seed crystals are typically added in an amount of 0.1 wt% to 10 wt%, preferably in an amount of 0.5 wt% to 7.0 wt%, most preferably 1.0 wt.% 5.0 wt%, on the basis of the total amount of the starting material used in step (i).

In step (iv) the mixture is stirred at a temperature in the range of from 15 to 30°C, preferably in the range of from 20 to 30°C and kept at this temperature, typically for a period of time from 2 to 24 hours, under stirring conditions or without stirring, in order to promote crystallization. After crystallization, the mixture can optionally be cooled to a temperature in the range of from 0 to 20°C, preferably in the range of from 5 to 15°C and/or one or more anti-solvents can be added to the mixture to increase the process yield. Regarding the anti-solvents, no specific restrictions exist, provided that the crystalline salt of betrixaban is not soluble and is stable.

Optionally, isolation in step (v) may be performed by using procedures known in the art, such as by filtration, centrifugation, or evaporation of solvent. Moreover, the isolated crystals may optionally be dried in step (vi), e.g. under reduced pressure, typically at room temperature, or heated up to a temperature between 25°C and 40°C or the crystals may directly be used in further processes.

### Process for the preparation of betrixaban hydrochloride form A

The present invention also relates to a process for the preparation of the crystalline form A of betrixaban hydrochloride according to the general process described above.

In step (iia) the solvent is selected from the group consisting of C₂ to C₄ alcohols and mixtures thereof, preferably from the group consisting of ethanol, *n*-propanol, isopropanol, *n*-butanol, 2-butanol, isobutanol and *tert*-butanol, more preferably the solvent is ethanol.

The mixture obtained in step (iia) may comprise about 20 to 400 g betrixaban per liter of solvent, preferably about 50 to 200 g betrixaban per liter of solvent.

In step (iib) hydrochloric acid, optionally diluted in the solvent used in step (iia), is slowly added to the mixture obtained in step (iia) under stirring conditions at a temperature in the range of from 10 to 40°C, more preferably in the range of from 20 to 30°C, preferably at ambient pressure.

### Process for the preparation of betrixaban hydrochloride form B

The present invention also relates to the preparation of the crystalline form B of betrixaban hydrochloride comprising the step of subjecting crystalline form A of betrixaban hydrochloride obtained or obtainable by the above-described process to thermal conditions leading to the polymorphic transformation of crystalline form A to crystalline form B of betrixaban hydrochloric. Polymorphic transformation of crystalline form A to crystalline form B occurs at a temperature in the range of from 230 to 250°C.

The crystalline form B of betrixaban hydrochloride obtained or obtainable by the above-described process can be used as seed crystals to crystallize form B directly from a solution according to the general process described above.

### Process for the preparation of betrixaban citrate

The present invention also relates to a process for the preparation of betrixaban citrate according to the general process described above.

In step (iia) the solvent is selected from the group consisting of C3-C6 ketones, C2-C4 alcohols, C3-C5 esters, and a combination of two or more thereof. Preferably the solvent is selected from the group consisting of acetone, cyclopentanone, diethyl ketone, methyl *n*-propyl ketone, methyl isopropyl ketone, methyl *n*-butyl ketone, methyl isobutyl ketone, methyl *tert*-butyl ketone, ethyl *n*-propyl ketone, ethyl isopropyl ketone, ethanol, *n*-propanol, isopropanol, *n-*butanol, 2-butanol, isobutanol, *tert*-butanol, methyl acetate, ethyl acetate, *n*-propyl acetate, isopropyl acetate, and mixtures thereof, more preferably from the group consisting of acetone and ethanol.

The mixture obtained in step (iia) may comprise about 20 to 400 g betrixaban per liter of solvent, preferably about 50 to 200 g betrixaban per liter of solvent. In step (iib) the addition of citric acid, may take place before or after the addition of the solvent in step (iia).

### Process for the preparation of betrixaban hydrobromide form B

The present invention also relates to a process for the preparation of the crystalline form B of betrixaban hydrobromide according to the general process described above.

In step (iia) the solvent is selected from the group consisting of acetonitrile, C3-C6 ketones, C2-C4 alcohols, C3-C5 esters, and a combination of two or more thereof. Preferably the solvent is selected from acetonitrile, acetone, cyclopentanone, diethyl ketone, methyl *n*-propyl ketone, methyl isopropyl ketone, methyl *n*-butyl ketone, methyl isobutyl ketone, methyl *tert*-butyl ketone, ethyl *n*-propyl ketone, ethyl isopropyl ketone, ethanol, *n*-propanol, isopropanol, *n-*butanol, 2-butanol, isobutanol, *tert*-butanol, methyl acetate, ethyl acetate, *n*-propyl acetate, and isopropyl acetate, more preferably from the group consisting of acetonitrile, acetone, ethanol and ethyl acetate.

The suspension obtained in step (iia) may comprise about 10 to 400 g betrixaban per liter solvent, preferably about 20 to 200 g betrixaban per liter solvent may be applied.

In step (iib) hydrobromic acid, optionally diluted in the at least one solvent used in step (iia), is slowly added to the suspension obtained in step (iia) under stirring conditions at a temperature in the range of from 10 to 40°C, more preferably in the range of from 20 to 30°C, preferably at ambient pressure.

### Pharmaceutical compositions and use

In a further aspect, the present invention relates to a pharmaceutical composition comprising the crystalline betrixaban salts of the invention and one or more pharmaceutically acceptable excipients.

The one or more pharmaceutically acceptable excipient(s) is/are preferably selected from the group consisting of carriers, fillers, diluents, lubricants, glidants, sweeteners, stabilizing agents, solubilizing agents, antioxidants and preservatives, flavouring agents, binders, colorants, osmotic agents, buffers, surfactants, disintegrants, granulating agents, coating materials and combinations thereof. In a preferred embodiment, the one or more pharmaceutically acceptable excipient(s) is/are selected from the group consisting of diluents, binders, disintegrants, lubricants and glidants.

The pharmaceutical composition of the present invention is preferably an oral solid dosage form, such as a tablet or a capsule. Preferably, the pharmaceutical composition of the present invention is a hard gelatine capsule.

Preferably, the pharmaceutical composition comprises an amount of 40, 60 or 80 mg of the crystalline betrixaban salts of the invention, calculated as betrixaban free base.

The pharmaceutical composition of the present invention can be prepared by wet or dry processing methods. In certain embodiments the pharmaceutical composition is prepared by wet processing methods, such as, but not limited to, wet granulation methods. Suitable wet granulation methods comprise high-shear granulation or fluid-bed granulation. In another embodiment the pharmaceutical composition is prepared by dry processing methods, such as, but not limited to, direct compression or dry granulation methods. An example of dry granulation is roller compaction. The pharmaceutical composition obtained by dry or wet processing methods may be compressed into tablets, encapsulated or metered into sachets. Preferably, the pharmaceutical composition is encapsulated.

In a further aspect, the present invention relates to the crystalline betrixaban salts of the invention or the pharmaceutical composition comprising the same as described above for use as a medicament.

In yet another aspect, the present invention relates to the crystalline betrixaban salts of the invention or the pharmaceutical composition comprising the same as described above for use in the prevention and the treatment of disease conditions characterized by undesired thrombosis.

### Advantages

The crystalline salts of betrixaban of the present invention can be prepared with a reliable and controllable process and are stable at relative humidity in the range of 0 to 95% and upon storage under accelerated conditions, i.e. the polymorphic forms are controllable and stable at high relative humidity and upon drying.

Moreover, the crystalline salts of betrixaban of the present invention have a higher water solubility than crystalline betrixaban maleate as shown in table 1.

**Table 1: solubility in water at 25°C**

| Salt | Betrixaban maleate | | Betrixaban hydrochloride, form A | Betrixaban hydrochloride, form B | Betrixaban citrate, form A | Betrixaban hydrobromide, form B |
|---|---|---|---|---|---|---|
| | form I | form II* | | | | |
| Solubility (mg/mL) | 2.7 | 2.5 | >5 | >3 | >3 | >3 |

| | | | | | | |
|---|---|---|---|---|---|---|
| * As reported in WO 2012/031017 | | | | | | |

Additionally, betrixaban salts prepared with "GRAS" acid such as hydrochloric acid or citric acid can be beneficial for the prevention and the long-term treatment of disease conditions characterized by undesired thrombosis.

Crystalline betrixaban hydrochloride and crystalline form B of betrixaban hydrobromide have also high thermal and thermodynamic stabilities (e.g. melting point or polymorphic transformation at a temperature above 230°C). Their thermal and thermodynamic stabilities are superior in respect to crystalline betrixaban maleate, which melts at a temperature below 215°C.

### EXAMPLES

### Powder X-ray diffraction

Powder X-ray diffraction (PXRD) was performed with a PANalytical X'Pert PRO diffractometer equipped with a theta/theta coupled goniometer in transmission geometry, Cu-Kα_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions.

### Fourier transform infrared

Fourier transform infrared (FTIR) spectra were recorded on an MKII Golden Gate™ Single Reflection Diamond ATR (attenuated total reflection) cell with a Bruker Tensor 27 FTIR spectrometer with 4 cm⁻¹ resolution at ambient conditions. To record a spectrum a spatula tip of a sample was applied to the surface of the diamond in powder form. Then the sample was pressed onto the diamond with a sapphire anvil and the spectrum was recorded. A spectrum of the clean diamond was used as background spectrum.

### Differential scanning calorimetry

Differential scanning calorimetry (DSC) was performed on a Mettler Polymer DSC R instrument. The sample was heated in a 40 microL aluminum pan with pierced aluminum lid from 25 to 300°C at a rate of 10°C/min. Nitrogen (purge rate 50 mL/min) was used as purge gas.

### Thermogravimetric analysis

Thermogravimetric analysis (TGA) was performed on a Mettler TGA/DSC 1 instrument. The sample was heated in a 100 microL aluminum pan closed with an aluminum lid. The lid was automatically pierced at the beginning of the measurement. The sample was heated from 25 to 300°C at a rate of 10°C/min. Nitrogen (purge rate 50 mL/min) was used as purge gas.

### Gravimetric moisture sorption/desorption

Gravimetric moisture sorption/desorption (GMSD) isotherms were recorded with an SPSx-1µ moisture sorption analyzer (ProUmid, Ulm). The measurement cycle was started at ambient relative humidity (RH) of 30%. Relative humidity was then decreased to 5% RH in 5% steps, followed by a further decrease to 3% RH and to 0% RH. For this isotherm, as black filled square with a white x inside is used in the respective figures. Afterwards RH was increased from 0% to 95% RH in a sorption cycle and decreased to 0 % in a desorption cycle in 5% steps. Finally RH was increased to 40% RH in 5% steps. As to the isotherm obtained by the last step, a black filled square with a white star inside is used as symbol in the respective figures.

The time per step was set to a minimum of 2 hours and a maximum of 6 hours. If an equilibrium condition with a constant mass of ± 0.01 % within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1°C.

### Determination of the moisture stability

Moisture stability at accelerated stability conditions was performed in a Memmert constant climate chamber HPP110. 50-70 mg of crystalline betrixaban hydrochloride were exposed to an atmosphere having a relative humidity of 75±1 % and a temperature of 40±0.5°C for a period of time as indicated in examples 1-1 and 2-2 and analysed via PXRD as described above.

### Determination of the solubility in water

Solubility values were estimated by a solvent addition technique at a temperature of 25°C. About 10 mg of crystalline betrixaban salt were weighed out into a vial. Water was added to the vial in aliquots of 500 µL and 200 µL until the salt was dissolved. In between the water additions the samples was sonicated for about 5 min, followed by shaking at 25°C for about 10 to 15 min. The estimated solubility values a reported in table 1.

### Example 1: form A of betrixaban hydrochloride

### Example 1-1

Crystalline betrixaban (199.4 mg, prepared according to the procedure disclosed in WO 2001/19788, example 266) was suspended in 4 mL of ethanol. Hydrochloric acid (56.9 µL, 37% aqueous, 1.3 eq.) was added dropwise to the suspension at room temperature under stirring conditions, allowing betrixaban dissolution. The clear solution was further stirred at room temperature. After a few minutes, solid crystallized out of the solution. The suspension was further stirred for 20 hours at room temperature. Afterwards, the solid material was collected by filtration and dried at room temperature under vacuum (about 30 mbar) for 18 hours to yield form A of betrixaban hydrochloride. Characteristic PXRD pattern of the obtained form A of betrixaban hydrochloride is shown in figure 1. The corresponding peak list is provided in table 2 below (relative peak intensities can vary due to e.g. inter-apparatus variability, sample crystallinity, sample preparation, etc.).

**Table 2**

| **position / ° 2-theta** | **relative intensity / %** | | **position / ° 2-theta** | **relative intensity / %** |
|---|---|---|---|---|
| 9.3 ± 0.2 | 29 | | 24.4 ± 0.2 | 45 |
| 11.6 ± 0.2 | 27 | | 25.0 ± 0.2 | 100 |
| 12.1 ± 0.2 | 28 | | 26.2 ± 0.2 | 7 |
| 13.8 ± 0.2 | 66 | | 26.5 ± 0.2 | 23 |
| 14.5 ± 0.2 | 97 | | 26.9 ± 0.2 | 18 |
| 16.4 ± 0.2 | 44 | | 27.4 ± 0.2 | 5 |
| 17.1 ± 0.2 | 5 | | 28.3 ± 0.2 | 5 |
| 18.8 ± 0.2 | 55 | | 28.6 ± 0.2 | 48 |
| 19.8 ± 0.2 | 7 | | 29.2 ± 0.2 | 6 |
| 20.2 ± 0.2 | 5 | | 30.6 ± 0.2 | 8 |
| 20.9 ± 0.2 | 5 | | 30.9 ± 0.2 | 8 |
| 22.6 ± 0.2 | 13 | | 31.4 ± 0.2 | 7 |
| 22.9 ± 0.2 | 26 | | 34.5 ± 0.2 | 5 |
| 23.5 ± 0.2 | 22 | | 36.2 ± 0.2 | 9 |
| 23.8 ± 0.2 | 42 | | | |

The resulting crystalline form A of betrixaban hydrochloride was subjected to a moisture stability test. The PXRD pattern of the material after 8 weeks upon storage at a temperature of 40°C and a relative humidity of 75% was essentially identical to the one shown in figure 1.

Representative FTIR spectrum of the obtained form A of betrixaban hydrochloride is shown in figure 2.

Representative DSC curve of the obtained form A of betrixaban hydrochloride is shown in figure 3 and shows a first endothermic event with an onset temperature of about 232°C, a peak maximum at about 244°C corresponding to the transformation of the crystalline form A to crystalline form B of betrixaban hydrochloride, followed by a melting endotherm with an onset temperature of about 272°C and a peak maximum at about 273°C.

Representative TGA curve of the obtained form A of betrixaban hydrochloride is shown in figure 4. It shows no significant weight loss from 25 to about 230°C and a weight loss of about 0.4% from about 230 to 255°C.

Representative gravimetric moisture sorption/desorption (GMSD) isotherms of the obtained form A of betrixaban hydrochloride are displayed in figure 5.

### Example 1-2

Crystalline betrixaban (200.1 mg, prepared according to the procedure disclosed in WO 2001/19788, example 266) was suspended in 3 mL of ethanol. Hydrochloric acid (56.9 µL, 37% aqueous, 1.3 eq.) was diluted in 1 mL ethanol and added dropwise to the betrixaban suspension at room temperature under stirring condition, allowing betrixaban dissolution. The clear solution was further stirred at room temperature. After a few minutes, solid crystallized out of the solution. The suspension was further stirred for 15 hours at room temperature. The solid material was collected by filtration, washed with ethanol, and dried at room temperature under vacuum (about 30 mbar) for 18 hours to yield 152.4 mg of form A of betrixaban hydrochloride. The PXRD pattern of the obtained form A of betrixaban hydrochloride was essentially identical to the one shown in figure 1.

### Example 2: form B of betrixaban hydrochloride

Form A of betrixaban hydrochloride (about 20 mg obtained according to example 1) was placed on a Mettler TGA/DSC 1 instrument and heated in an open 100 microL aluminum pan from 25 to 250°C at a rate of 10°C/min and kept at 250°C for 5 minutes, yielding form B of betrixaban hydrochloride. Nitrogen (purge rate 50 mL/min) was used as purge gas. Characteristic PXRD pattern of the obtained form B of betrixaban hydrochloride is shown in figure 6. The corresponding peak list is provided in table 3 below (relative peak intensities can vary due to e.g. inter-apparatus variability, sample crystallinity, sample preparation, etc.).

**Table 3**

| **position / ° 2-theta** | **relative intensity / %** | | **position / ° 2-theta** | **relative intensity** / **%** |
|---|---|---|---|---|
| 9.0 ± 0.2 | 26 | | 22.8 ± 0.2 | 43 |
| 10.7 ± 0.2 | 6 | | 23.2 ± 0.2 | 23 |
| 11.6 ± 0.2 | 5 | | 23.4 ± 0.2 | 17 |
| 12.2 ± 0.2 | 5 | | 24.4 ± 0.2 | 23 |
| 12.5 ± 0.2 | 12 | | 25.6 ± 0.2 | 9 |
| 12.7 ± 0.2 | 25 | | 26.0 ± 0.2 | 6 |
| 14.5 ± 0.2 | 14 | | 26.3 ± 0.2 | 14 |
| 14.7 ± 0.2 | 100 | | 26.5 ± 0.2 | 11 |
| 14.9 ± 0.2 | 37 | | 27.7 ± 0.2 | 13 |
| 17.4 ± 0.2 | 24 | | 28.0 ± 0.2 | 8 |
| 18.1 ± 0.2 | 37 | | 29.9 ± 0.2 | 8 |
| 19.0 ± 0.2 | 17 | | 30.6 ± 0.2 | 8 |
| 19.8 ± 0.2 | 30 | | 31.3 ± 0.2 | 5 |
| 20.2 ± 0.2 | 6 | | 32.2 ± 0.2 | 5 |
| 21.3 ± 0.2 | 7 | | 35.4 ± 0.2 | 18 |
| 21.5 ± 0.2 | 7 | | | |

The resulting crystalline form B of betrixaban hydrochloride was subjected to a moisture stability test. The PXRD pattern of the material after 4 weeks upon storage at a temperature of 40°C and a relative humidity of 75% was essentially identical to the one shown in figure 6.

### Reference example 1: preparation of betrixaban hydrochloride according to example 4 of WO 2007/056517

### Reference example 1-1

50 mg of betrixaban free base (prepared according to the procedure disclosed in WO 2001/19788, example 266) were suspended in 7.5 mL of 10% (aq.) THF. 14.3 µL (1.1 eq.) of 37% aqueous hydrochloric acid in 2.5 mL ethanol were added. The mixture was shaken for 2 hours, followed by addition of 5 mL of *tert*-butyl methyl ether, yielding a biphasic solution. The mixture was further shaken for several days (up to 14 days) at room temperature. No precipitation was observed.

### Reference example 1-2 (under stirring conditions)

50 mg of betrixaban free base (prepared according to the procedure disclosed in WO 2001/19788, example 266) were suspended in 7.5 mL of 10% (aq.) THF. 14.3 µL (1.1 eq.) of 37% aqueous hydrochloric acid in 2.5 mL ethanol were added. The mixture was stirred for 2 hours, followed by addition of 5 mL of *tert*-butyl methyl ether, yielding a biphasic solution. The mixture was further stirred for several days (up to 14 days) at room temperature. No precipitation was observed.

The solution was cooled to 5°C and kept at this temperature for 2 days. No precipitation was observed.

### Reference example 1-3 (under stirring conditions, without tert-butyl methyl ether)

50 mg of betrixaban free base (prepared according to the procedure disclosed in WO 2001/19788, example 266) were suspended in 7.5 mL of 10% (aq.) THF. 14.3 µL (1.1 eq.) of 37% aqueous hydrochloric acid in 2.5 mL ethanol were added. The mixture was further stirred for several days (up to 14 days) at room temperature. No precipitation was observed.

The solution was open to allow slow solvent evaporation and further stirred under these conditions at room temperature for 2 days. No precipitation was observed.

### Example 3: betrixaban citrate (Form A)

### Example 3-1

Crystalline betrixaban (200.0 mg, prepared according to the procedure disclosed in WO 2001/19788, example 266) and citric acid (89.3 mg, 1.05 eq.) were suspended in 4 mL of acetone. The slurry was stirred for 22 hours at room temperature. Afterwards, the solid material was collected by filtration, washed with acetone and dried at room temperature under vacuum (about 30 mbar) for 18 hours to yield 187.7 mg of crystalline form A of betrixaban citrate. Characteristic PXRD pattern of the obtained crystalline betrixaban citrate is shown in figure 7. The corresponding peak list is provided in table 4 below (relative peak intensities can vary due to e.g. inter-apparatus variability, sample crystallinity, sample preparation, etc.).

**Table 4**

| **position / ° 2-theta** | **relative intensity / %** | | **position / ° 2-theta** | **relative intensity** / **%** |
|---|---|---|---|---|
| 6.2 ± 0.2 | 39 | | 23.6 ± 0.2 | 50 |
| 7.1 ± 0.2 | 31 | | 24.3 ± 0.2 | 20 |
| 8.1 ± 0.2 | 37 | | 24.9 ± 0.2 | 16 |
| 10.6 ± 0.2 | 13 | | 25.2 ± 0.2 | 35 |
| 12.7 ± 0.2 | 40 | | 25.7 ± 0.2 | 100 |
| 13.1 ± 0.2 | 35 | | 26.5 ± 0.2 | 19 |
| 13.7 ± 0.2 | 12 | | 27.5 ± 0.2 | 8 |
| 14.7 ± 0.2 | 80 | | 28.0 ± 0.2 | 9 |
| 15.9 ± 0.2 | 45 | | 28.4 ± 0.2 | 20 |
| 17.0 ± 0.2 | 74 | | 29.3 ± 0.2 | 19 |
| 18.1 ± 0.2 | 72 | | 31.1 ± 0.2 | 15 |
| 18.3 ± 0.2 | 69 | | 32.1 ± 0.2 | 14 |
| 19.7 ± 0.2 | 28 | | 33.0 ± 0.2 | 16 |
| 20.6 ± 0.2 | 78 | | 33.8 ± 0.2 | 7 |
| 21.1 ± 0.2 | 39 | | 35.3 ± 0.2 | 6 |
| 21.4 ± 0.2 | 23 | | 36.6 ± 0.2 | 10 |
| 21.7 ± 0.2 | 22 | | 38.3 ± 0.2 | 6 |
| 22.8 ± 0.2 | 23 | | 39.0 ± 0.2 | 5 |
| 23.3 ± 0.2 | 44 | | | |

The resulting crystalline form of betrixaban citrate was subjected to a moisture stability test. The PXRD pattern of the material after 8 weeks upon storage at a temperature of 40°C and a relative humidity of 75% was essentially identical to the one shown in figure 7.

Representative DSC curve of the obtained crystalline form A of betrixaban citrate shows a melting endotherm with an onset temperature of about 166°C and a peak maximum at about 168°C.

Representative TGA curve of the obtained crystalline form A of betrixaban citrate shows a continuous weight loss of less than 1.0% from 25 to 166°C, corresponding to non-bounded residual solvent. No modification of the physical form of crystalline form A of betrixaban citrate is observed from 25 to 166°C.

Representative gravimetric moisture sorption/desorption (GMSD) isotherms of the obtained crystalline form A of betrixaban citrate are displayed in figure 8.

### Example 3-2

Crystalline betrixaban (100.3 mg, prepared according to the procedure disclosed in WO 2001/19788, example 266) and citric acid (44.6 mg, 1.05 eq.) were suspended in 2 mL of ethanol. The slurry was stirred for 18 hours at room temperature. Afterwards, the solid material was collected by filtration, washed with ethanol, and dried at room temperature under vacuum (about 30 mbar) for 15 hours to yield crystalline form A of betrixaban citrate. The PXRD pattern of the obtained crystalline form of betrixaban citrate was essentially identical to the one shown in figure 7.

### Example 4: form B of betrixaban hydrobromide

### Example 4-1

Crystalline betrixaban (200.5 mg, prepared according to the procedure disclosed in WO 2001/19788, example 266) was suspended in 10 mL of acetonitrile. Hydrobromic acid (54.0 µL, 47% aqueous, 1.05 eq.) was added dropwise to the suspension at room temperature under stirring conditions, leading to the formation of a yellow suspension. The slurry was further stirred for 18 hours at room temperature. Afterwards, the solid material was collected by filtration, washed with acetonitrile, and dried at room temperature under vacuum (about 30 mbar) for 20 hours to yield 204.5 mg of crystalline form B of betrixaban hydrobromide. Characteristic PXRD pattern of the obtained crystalline form of betrixaban hydrobromide is shown in figure 9. The corresponding peak list is provided in table 5 below (relative peak intensities can vary due to e.g. inter-apparatus variability, sample crystallinity, sample preparation, etc.).

**Table 5**

| **position / ° 2-theta** | **relative intensity / %** | | **position / ° 2-theta** | **relative intensity** / **%** |
|---|---|---|---|---|
| 11.6 ± 0.2 | 10 | | 24.6 ± 0.2 | 100 |
| 12.5 ± 0.2 | 35 | | 25.0 ± 0.2 | 16 |
| 13.6 ± 0.2 | 27 | | 25.3 ± 0.2 | 14 |
| 13.7 ± 0.2 | 22 | | 26.1 ± 0.2 | 7 |
| 15.0 ± 0.2 | 28 | | 26.6 ± 0.2 | 12 |
| 16.0 ± 0.2 | 55 | | 27.2 ± 0.2 | 9 |
| 16.6 ± 0.2 | 22 | | 27.6 ± 0.2 | 5 |
| 17.5 ± 0.2 | 8 | | 28.1 ± 0.2 | 13 |
| 18.8 ± 0.2 | 45 | | 28.5 ± 0.2 | 30 |
| 20.4 ± 0.2 | 22 | | 29.5 ± 0.2 | 5 |
| 21.1 ± 0.2 | 5 | | 30.3 ± 0.2 | 15 |
| 22.0 ± 0.2 | 21 | | 31.8 ± 0.2 | 7 |
| 23.0 ± 0.2 | 28 | | 32.3 ± 0.2 | 5 |
| 23.5 ± 0.2 | 25 | | 33.4 ± 0.2 | 13 |
| 23.8 ± 0.2 | 37 | | 36.0 ± 0.2 | 7 |

The resulting crystalline form of betrixaban hydrobromide was subjected to a moisture stability test. The PXRD pattern of the material after 8 weeks upon storage at a temperature of 40 °C and a relative humidity of 75% was essentially identical to the one shown in figure 9.

Representative DSC curve of the obtained the crystalline form B of betrixaban hydrobromide shows a melting endotherm with an onset temperature of about 246°C and a peak maximum at about 249°C.

Representative TGA curve of the obtained crystalline form B of betrixaban hydrobromide shows a continuous weight loss of less than 1.5% from 25 to 246°C, corresponding to non-bounded residual solvent. No modification of the physical form of crystalline form B of betrixaban hydrobromide is observed from 25 to 246°C.

Representative gravimetric moisture sorption/desorption (GMSD) isotherms of the obtained form B of betrixaban hydrobromide are displayed in figure 10.

### Example 4-2

Crystalline betrixaban (200.0 mg, prepared according to the procedure disclosed in WO 2001/19788, example 266) was suspended in 9 mL of acetonitrile. Hydrobromic acid (54.0 µL, 47% aqueous, 1.05 eq.) was diluted in 1 mL acetonitrile. The acidic solution was added dropwise to the betrixaban suspension at room temperature under stirring conditions, leading to the formation of a yellow suspension. The slurry was further stirred for 16 hours at room temperature. Afterwards, the solid material was collected by filtration, washed with acetonitrile, and dried at room temperature under vacuum (about 30 mbar) for 22 hours to yield 195.5 mg of crystalline form B of betrixaban hydrobromide. The PXRD pattern of the obtained crystalline form of betrixaban hydrobromide was essentially identical to the one shown in figure 9.

### Example 4-3

Crystalline betrixaban (100.0 mg, prepared according to the procedure disclosed in WO 2001/19788, example 266) was suspended in 2 mL of acetone. Hydrobromic acid (27.0 µL, 47% aqueous, 1.05 eq.) was added dropwise to the suspension at room temperature under stirring conditions, leading to the formation of a thick yellow suspension. To allow a better stirring 2 mL of acetone were added to the slurry, which was further stirred for 24 hours at room temperature. Afterwards, the solid material was collected by filtration, washed with acetone, and dried at room temperature under vacuum (about 30 mbar) for 20 hours to yield crystalline form B of betrixaban hydrobromide. The PXRD pattern of the obtained crystalline form of betrixaban hydrobromide was essentially identical to the one shown in figure 9.

### Example 4-4

Crystalline betrixaban (100.2 mg, prepared according to the procedure disclosed in WO 2001/19788, example 266) was suspended in 2 mL of ethyl acetate. Hydrobromic acid (27.0 µL, 47% aqueous, 1.05 eq.) was added dropwise to the suspension at room temperature under stirring conditions, leading to the formation of a thick yellow suspension. To allow a better stirring 2 mL of ethyl acetate were added to the slurry, which was further stirred for 24 hours at room temperature. Afterwards, the solid material was collected by filtration, washed with ethyl acetate, and dried at room temperature under vacuum (about 30 mbar) for 20 hours to yield crystalline form B of betrixaban hydrobromide. The PXRD pattern of the obtained crystalline form of betrixaban hydrobromide was essentially identical to the one shown in figure 9.

### Example 4-5

Crystalline betrixaban (100.1 mg, prepared according to the procedure disclosed in WO 2001/19788, example 266) was suspended in 2 mL of ethanol. Hydrobromic acid (27.0 µL, 47% aqueous, 1.05 eq.) was added dropwise to the suspension at room temperature under stirring conditions, leading to the formation of a thick yellow suspension. To allow a better stirring 2 mL of ethanol were added to the slurry, which was further stirred for 24 hours at room temperature. Afterwards, the solid material was collected by filtration, washed with ethanol, and dried at room temperature under vacuum (about 30 mbar) for 20 hours to yield crystalline form B of betrixaban hydrobromide. The PXRD pattern of the obtained crystalline form of betrixaban hydrobromide was essentially identical to the one shown in figure 9.

## Claims

1. Crystalline betrixaban hydrochloride.

2. Crystalline betrixaban hydrochloride according to claim 1, **characterized by** having a powder X-ray diffractogram comprising reflections at 2-Theta angles of 9.3 ± 0.2°, 12.1 ± 0.2°, 13.8 ± 0.2°, 14.5 ± 0.2° and 25.0 ± 0.2°, when measured at room temperature using Cu-Kα_{1,2} radiation having a wavelength of 0.15419 nm.

3. Crystalline betrixaban hydrochloride according to claim 2, wherein the powder X-ray diffractogram comprises an additional reflection at one or more 2-Theta angle(s) of 11.6 ± 0.2°, 16.4 ± 0.2°, 18.8 ± 0.2°, 22.6 ± 0.2°, 22.9 ± 0.2°, 23.5 ± 0.2°, 23.8 ± 0.2°, 24.4 ± 0.2°, 26.9 ± 0.2° and/or 28.6 ± 0.2°, when measured at room temperature with Cu-Kα_{1,2} radiation having a wavelength of 0.15419 nm.

4. Crystalline betrixaban hydrochloride according to any one of the preceding claims having a DSC curve comprising a first endothermic event with an onset temperature of about 232°C and a peak maximum at about 244°C, followed by a second endotherm with an onset temperature of about 272°C and a peak maximum at about 273°C, when measured at a heating rate of 10°C/min.

5. Crystalline betrixaban hydrochloride according to claim 1, **characterized by** having a powder X-ray diffractogram comprising reflections at 2-Theta angles of 9.0 ± 0.2°, 12.7 ± 0.2°, 14.7 ± 0.2°, 18.1 ± 0.2° and 22.8 ± 0.2°, when measured at room temperature using Cu-Kα_{1,2} radiation having a wavelength of 0.15419 nm.

6. Crystalline betrixaban hydrochloride according to claim 5, wherein the powder X-ray diffractogram comprises an additional reflection at one or more 2-Theta angle(s) of 12.5 ± 0.2°, 14.5 ± 0.2°, 14.9 ± 0.2°, 17.4 ± 0.2°, 19.0 ± 0.2°, 19.8 ± 0.2°, 23.2 ± 0.2°, 23.4 ± 0.2°, 24.4 ± 0.2°, 26.3 ± 0.2°, 26.5 ± 0.2° and/or 27.7 ± 0.2°, when measured at room temperature with Cu-Kα_{1,2} radiation having a wavelength of 0.15419 nm.

7. Crystalline betrixaban hydrochloride according to claims 1, 5 or 6 having a melting point of from about 272 to 273°C when measured with DSC at a heating rate of 10°C/min.

8. Crystalline betrixaban citrate.

9. Crystalline betrixaban citrate according to claim 8, **characterized by** having a powder X-ray diffractogram comprising reflections at 2-Theta angles of 6.2 ± 0.2°, 8.1 ± 0.2°, 12.7 ± 0.2°, 14.7 ± 0.2° and 25.7 ± 0.2°, when measured at room temperature using Cu-Kα_{1,2} radiation having a wavelength of 0.15419 nm.

10. Crystalline betrixaban citrate according to claim 9, wherein the powder X-ray diffractogram comprises an additional reflection at one or more 2-Theta angle(s) of 7.1 ± 0.2°, 13.1 ± 0.2°, 15.9 ± 0.2°, 17.0 ± 0.2°, 18.1 ± 0.2°, 18.3 ± 0.2°, 20.6 ± 0.2°, 21.1 ± 0.2°, 23.3 ± 0.2°, 23.6 ± 0.2° and/or 25.2 ± 0.2°, when measured at room temperature with Cu-Kα_{1,2} radiation having a wavelength of 0.15419 nm.

11. Crystalline betrixaban citrate according to according to claims 8 to 10, having a DSC curve comprising an endotherm with an onset temperature of about 166°C and a peak maximum at about 168°C when measured at a heating rate of 10°C/min.

12. Crystalline betrixaban hydrobromide **characterized by** having a powder X-ray diffractogram comprising reflections at 2-Theta angles of 12.5 ± 0.2°, 13.6 ± 0.2°, 16.0 ± 0.2°, 18.8 ± 0.2° and 24.6 ± 0.2°, when measured at room temperature using Cu-Kα_{1,2} radiation having a wavelength of 0.15419 nm.

13. Crystalline betrixaban hydrobromide according to claim 12, wherein the powder X-ray diffractogram comprises an additional reflection at one or more 2-Theta angle(s) 11.6 ± 0.2°, 13.7 ± 0.2°, 15.0 ± 0.2°, 16.6 ± 0.2°, 20.4 ± 0.2°, 22.0 ± 0.2°, 23.0 ± 0.2°, 23.5 ± 0.2°, 23.8 ± 0.2°, 25.0 ± 0.2° and/ or 25.3 ± 0.2°, when measured at room temperature with Cu-Kα_{1,2} radiation having a wavelength of 0.15419 nm.

14. A pharmaceutical composition comprising the betrixaban salts according to claims 1 to 13 and one or more pharmaceutically acceptable excipients.

15. Crystalline betrixaban salts according to claims 1 to 13 or the pharmaceutical composition of claim 14 for use as a medicament.
